# EUROPÄISCHE PATENTSCHRIFT

(11) **EP 4 129 191 B1**
(45) Veröffentlichungstag und Bekanntmachung des Hinweises auf die Patenterteilung: **04.03.2026**
(21) Anmeldenummer: 21190056.8
(22) Anmeldetag: 06.08.2021
(51) Int. Cl.: A61B 6/00, A61B 6/40, A61B 6/51

(54) **OPTIMIERUNG EXTRAORALER PANORAMAAUFNAHMEN DURCH MODELLBASIERTES VORWISSEN ÜBER DIE KIEFERBOGENFORM DES PATIENTEN**
OPTIMIZATION OF EXTRAORAL PANORAMA IMAGES BY MODEL-BASED PRE-KNOWLEDGE ABOUT THE SHAPE OF THE PATIENT'S JAW ARCH
OPTIMISATION DES ENREGISTREMENTS PANORAMIQUES EXTRA-BUCCAUX AU MOYEN DES CONNAISSANCES ANTÉRIEURES BASÉES SUR LE MODÈLE DE LA FORME DE L'ARC MANDIBULAIRE DU PATIENT

(43) Veröffentlichungstag der Anmeldung: 08.02.2023
(73) Patentinhaber: DENTSPLY SIRONA Inc., York, PA 17401 (US); Sirona Dental Systems GmbH, 64625 Bensheim (DE)
(72) Erfinder: Eichner, Stefan, 64625 Bensheim (DE); Elvers, Michael, 64625 Bensheim (DE)
(74) Vertreter: Taor, Simon Edward William

(56) Entgegenhaltungen:
- EP-A1- 2 465 436
- EP-A1- 2 614 773
- EP-A1- 3 711 672
- WO-A1-2019/002631
- WO-A1-2019/063797

## Beschreibung

### TECHNISCHES GEBIET DER ERFINDUNG

Die vorliegende Erfindung bezieht sich auf Verfahren zur Herstellung digitaler Panoramaaufnahmen mittels extraoraler Röntgengeräte .

### HINTERGRUND DER ERFINDUNG

Bei der Herstellung von einer Panoramaaufnahme, bildet die Standard-Bahnkurve (Default-Bahnkurve) eines extraoralen Röntgengerätes eine empirisch gefundene SOLL-Fokalkurve im Patientenkiefer ab. Im Falle einer Abweichung von dieser Referenz, sei es durch eine Fehlpositionierung oder eine vom Modell abweichende Kieferbogenform (IST-Fokalkurve), kommt es zu einer *verschlechterten* Panorama-Bildqualität aufgrund einer nicht optimalen Bahnkurve und/oder rekonstruierten Fokalkurvenlage.

Ein nachgelagerter Autofokus versucht die optimale Schärfe lokal innerhalb der Panoramaaufnahme zu finden. Starke anatomische Anomalien und/oder ungünstige Durchstrahlwinkel können damit nicht korrigiert werden. Hierbei handelt es sich immer um nachgelagerte Software-Lösungen, so dass eine aufwendigere Rekonstruktion notwendig wird, die nur die erzeugten Daten basierend auf der Default-Bahnkurve nutzen kann.

WO2019/063797 offenbart ein Verfahren und System zur Generierung eines Panoramabildes.

WO2019/002631 offenbart die Klassifizierung und 3D-Modellierung von 3D-Zahn-, Kiefer- und Gesichtspatiestrukturen mit tiefenlernverfahren.

### OFFENBARUNG DER ERFINDUNG

Ein Ziel der vorliegenden Erfindung ist die Bereitstellung eines Verfahren zur Herstellung digitaler Panoramaaufnahmen mittels extraoraler Röntgengeräte mit dem die oben genannten Nachteile des Standes der Technik überwinden werden kann.

Diese Ziele werden durch das Verfahren nach Anspruch 1 erreicht. Die Gegenstände der abhängigen Ansprüche beziehen sich auf Weiterentwicklungen.

Die vorliegende Erfindung bietet ein Verfahren zur Herstellung digitaler Panoramaaufnahmen mittels extraoraler Röntgengeräte. Es umfasst folgenden Schritte: Basierend auf einer oder mehrerer vorangegangener Panoramaaufnahmen oder 3D-Aufnahmen oder eines oder mehrerer optischen 3D-Scans eines Patienten, wird die Kieferbogenform als modellbasiertes Vorwissen über die Anatomie des Patienten ermittelt; Verwendung des modellbasierten Vorwissens um eine patienten-spezifische Röntgengerätebahnkurve der herzustellenden Panoramaaufnahme des Patienten zu ermitteln sodass die Kieferbogenform bzw. die Stellung der Zähne optimal aufgenommen werden kann; Durchführen der Aufnahme auf Basis der ermittelten patienten-spezifischen Röntgengerätebahnkurve; Anpassung von Rekonstruktionsparametern entsprechend der ermittelten patienten-spezifischen Röntgengerätebahnkurve; Verwendung des modellbasierten Vorwissens, um die patienten-spezifischen Fokalkurve der herzustellenden Panoramaaufnahme des Patienten zu ermitteln sodass die Kieferbogenform bzw. die Stellung der Zähne optimal rekonstruiert werden kann; Rekonstruktion der Panoramaaufnahme unter Verwendung der Aufnahmedaten, der angepassten Rekonstruktionsparameter einschließlich der ermittelten patienten-spezifischen Fokalkurve; Darstellung der rekonstruierten Panoramaaufnahme.

Ein vorteilhafter Effekt der vorliegenden Erfindung ist, dass sie in Summe aufgrund der verbesserten Panorama-Bildqualität einen diagnostischen Mehrwert für den Arzt schafft. Wiederholungsaufnahmen und zusätzliche Scout-Schüsse werden vermieden. Die Dosis kann reduziert bzw. die Bildqualität verbessert werden.

Ein weiterer vorteilhafter Effekt der vorliegenden Erfindung ist, dass für einen Patienten anhand einer vorangegangenen Panoramaaufnahme, 3D Volumenaufnahme oder Oberflächenscan die Kieferbogenform bzw. Kieferbogengeometrie abgeleitet werden kann um die passende Bahnkurve und passende Fokalkurve zu ermitteln sodass die Panoramaaufnahme, sowie die Rekonstruktion patientenspezifisch erfolgen kann. Hierbei besteht die Möglichkeit einer partiellen patientenspezifischen Bahnkurve/Rekonstruktion für die Erstellung von Teilausschnitten einer Panoramaaufnahme zu verwenden.

Es werden ein oder mehrere neuronale Netze benutzt. Die Kieferbogenform kann mittels neuronaler Netze ermittelt werden. Die neuronalen Netze werden durch Datenpaare trainiert, welche 3D Volumen und die darin markierte Kieferbogenform umfassen, oder den optischen 3D-Scan und darin markierte Kieferbogenform umfassen. Hierzu kann die Kieferbogenform manuell oder durch Bildverarbeitung unter Berücksichtigung anatomischer Merkmale automatisch markiert worden sein.

In einer vorteilhaften Ausführungsform bei der Rekonstruktion der Panoramaaufnahme, ist der Überlapp des Kieferbogens und der scharfen Schicht maximal, wobei die patienten-spezifische Fokalkurve innerhalb der scharfen Schicht liegt und bestmöglich den Kieferbogen abbildet.

Die Erfindung stellt auch ein computergestütztes extraorales Röntgensystem bereit, um das Verfahren durchzuführen. Das extraorale Röntgensystem kann die rekonstruierte Panoramaaufnahme bereitstellen, wobei die Rechenschritte auf demselben extraoralen Röntgensystem oder auf einem separaten Rechner bzw. in der Cloud durchgeführt werden können.

### KURZBESCHREIBUNG DER ZEICHNUNGEN

In der nachfolgenden Beschreibung wird die vorliegende Erfindung anhand von beispielhaften Ausführungsformen und unter Bezugnahme auf die Zeichnungen näher erläutert, wobei
Abb.1 - zeigt eine schematische Darstellung eines extraoralen Röntgensystems nach einer Ausführungsform;
Abb.2 - zeigt eine schematische Darstellung einer extraoralen Panoramaaufnahme gemäß der ermittelten patienten-spezifischen Bahnkurve.

Die in den Zeichnungen gezeigten Referenznummern bezeichnen die unten aufgeführten Elemente, auf die in der nachfolgenden Beschreibung der beispielhaften Ausführungsformen Bezug genommen wird.
- 1.: Extraorales Röntgensystem
- 2.: Röntgengerät
- 3.: Röntgenstrahler
- 4.: Röntgendetektor
- 5.: Bedienungseinheit
- 6.: Kopffixierung
- 7.: Aufbiss
- 8.: Rechner
- 9.: Anzeige
- 10.: Patient
- 11a.: Kieferbogenform
- 11b.: Zahn
- 12.: Bahnkurve (Patienten-spezifisch)
- 12a.: Röntgenstrahler-Trajektorie
- 12b.: Röntgendetektor-Trajektorie
- 13.: Bahnkurve (Röntgengeräte-Default)
- 13a.: Röntgenstrahler-Trajektorie
- 13b.: Röntgendetektor-Trajektorie
- 14.: Fokalkurve (Patienten-spezifisch)
- 15.: Fokalkurve (Röntgengeräte-Default)

Das erfindungsgemäße Verfahren dient zur Herstellung einer Panoramaaufnahme.

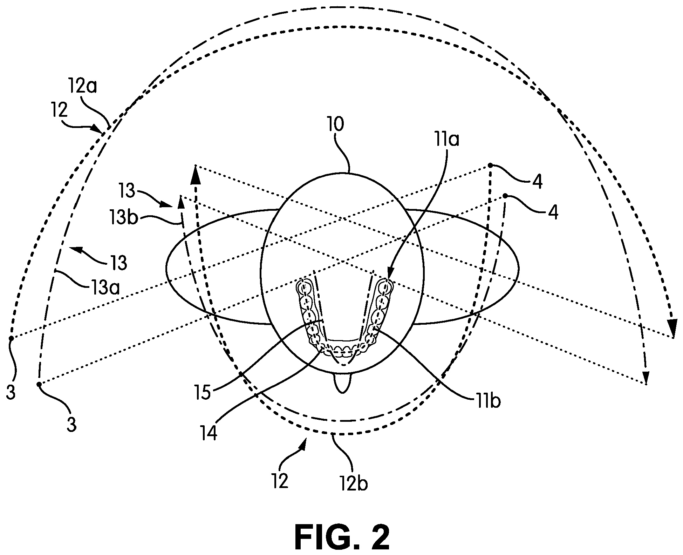

In einem ersten Schritt S1 wird basierend auf einer oder mehrere vorangegangenen Panoramaaufnahmen oder 3D-Aufnahmen oder eines oder mehrerer optischer 3D-Scans eines Patienten, die Kieferbogenform (11a) (s. Abb.2) als modellbasiertes Vorwissen über die Anatomie des Patienten (10) ermittelt. Dieses Vorwissen kann optional zwischengespeichert und für spätere Verwendung abgerufen werden. In einem weiteren Schritt S2 wird das modellbasierte Vorwissens verwendet um eine patienten-spezifischen Bahnkurve (12) (s. Abb. 2) der herzustellenden Panoramaaufnahme des Patienten (10) zu ermitteln sodass die Kieferbogenform (11a) bzw. die Stellung der Zähne (11b) optimal aufgenommen werden kann. Die patienten-spezifische Bahnkurve (12) besteht aus der patienten-spezifischen Röntgenstrahler-Trajektorie (12a) und der patienten-spezifischen Röntgendetektor-Trajektorie (12b). Wie Abb.2 zeigt, wird in einem weiteren Schritt S3 die Aufnahme auf Basis der ermittelten patienten-spezifischen Bahnkurve (12) durchgeführt.

Zum Vergleich wurde in Abb. 2 auch die Röntgengeräte-Default-Bahnkurve (13) dargestellt. Wobei die Röntgengeräte-Default-Bahnkurve (13) aus der Röntgengeräte-Default-Röntgenstrahler-Trajektorie (13a) und der Röntgengeräte-Default-Röntgendetektor-Trajektorie (13b) besteht. In einem weiteren Schritt S4 werden die Rekonstruktionsparametern entsprechend der ermittelten patienten-spezifischen Bahnkurve (12) angepasst. In einem weiteren Schritt S5 wird das modellbasierten Vorwissens verwendet, um die patienten-spezifischen Fokalkurve (14) (s.Abb.2) der herzustellenden Panoramaaufnahme des Patienten (10) zu ermitteln sodass die Kieferbogenform (11a) bzw. die Stellung der Zähne (11b) optimal rekonstruiert werden kann. Zum Vergleich wurde in Abb. 2 auch die Röntgengeräte-Default-Fokalkurve (15) gezeigt. In einem weiteren Schritt S6 wird eine Panoramaaufnahme rekonstruiert unter Verwendung der Aufnahmedaten, der angepassten Rekonstruktionsparameter einschließlich der ermittelten patienten-spezifischen Fokalkurve (14). Die patienten-spezifische Fokalkurve (14) liegt innerhalb einer scharfen Schicht. Der Überlapp der Kieferbogenform (11a) und der scharfen Schicht ist vorzugsweise maximal. In einem weiteren Schritt S7 wird die rekonstruierte Panoramaaufnahme dargestellt.

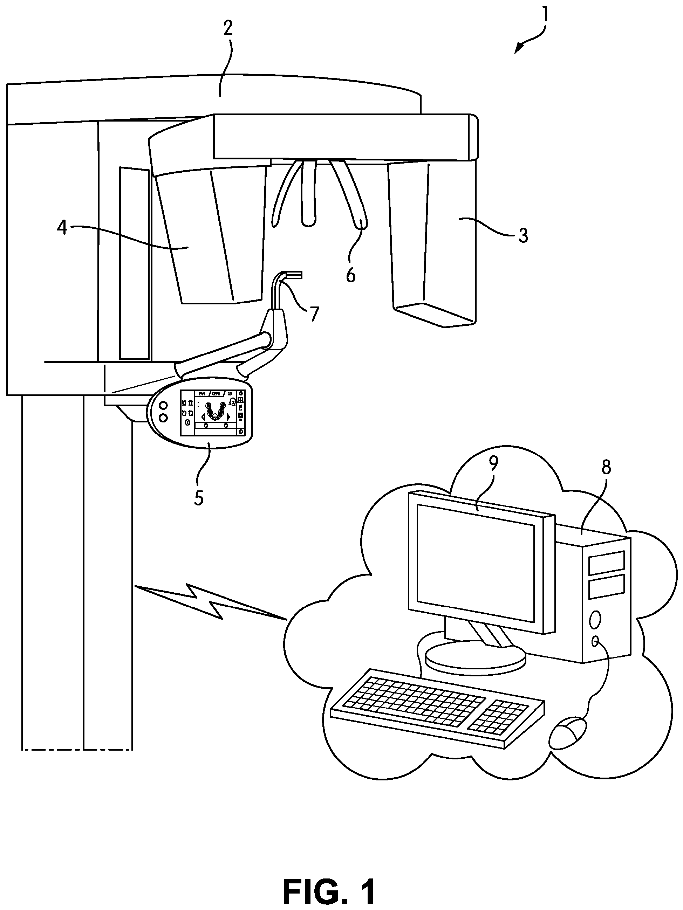

Das erfindungsgemäße Verfahren ist ein Computer implementiertes Verfahren, und kann auf einem computergestützten extraorales Röntgensystem (1) ausgeführt werden. Abb. 1 zeigt ein Ausführungsbespiel des extraorales Röntgensystems (1). Das erfindungsgemäße Verfahren wird durch ein Computerprogramm implementiert, welches computerlesbaren Code aufweist. Das Computerprogramm kann auf einem Datenspeicher bereitgestellt werden. Wie in Abb. 1 gezeigt, umfasst das computergestützte extraorale Röntgen -System (1) ein Röntgengerät (2) zur Durchführung der Patientenaufnahme, womit einzelne 2D Röntgenbilder erzeugt werden. Das Röntgengerät (2) hat einen Röntgenstrahler (3) und Röntgendetektor (4), die während der Aufnahme um den Patiententkopf gedreht werden. Das Röntgengerät (2) hat einen Drehmechanismus, wodurch der Röntgenstrahler (3) und der Röntgendetektor (4) um den Patiententkopf gemäß der ermittelten patienten-spezifischen Bahnkurve (12) oder alternativ gemäß der Röntgengeräte-Default-Bahnkurve (13) gedreht werden kann. Der Drehmechanismus ermöglicht das Abfahren der patienten-spezifischen Bahnkurve (12). Das Röntgengerät (2) kann auch so angesteuert werden, dass die Default-Bahnkurve für das Röntgengerät (13) abgefahren wird. Die Bahnkurven (12;13) können eine Kreisbahn beschreiben. Alternativ können sie eine davon abweichende komplexere Kurvenform annehmen. Vor der Aufnahme wird der Patientenkopf mit dem Aufbiss (7) und der Kopffixierung (6) im Röntgengerät (2) positioniert. Wie in Abb. 1 gezeigt hat das computergestützte extraorale Röntgensystem (1) eine Bedienungseinheit (5), vorzugsweise einen separaten Rechner (8) oder eine Recheneinheit, die mit dem Röntgengerät (2) verbunden werden kann, und vorzugsweise eine separate Anzeige (9), u.a. um Datensätze zu visualisieren. Der Rechner (8) kann über ein lokales Netzwerk (nicht gezeigt) oder alternativ über das Internet mit dem Röntgengerät (2) verbunden werden. Der Rechner (8) kann Teil einer Cloud sein. Alternativ kann der Rechner (8) in das Röntgengerät (2) integriert werden. Die Berechnungen können im Rechner (8) bzw. in der Cloud stattfinden. Dazu können die Rohdaten komprimiert übertragen werden. Der Rechner (8) führt das Computerprogramm aus und liefert die Datensätze, u.a. auch für die Visulisierung auf der Anzeige (9). Die Anzeige (9) kann räumlich von dem Röntgengerät (2) getrennt sein. Der Rechner (8) kann vorzugsweise auch das Röntgengerät (2) steuern. Alternativ können separate Rechner (8) für die Steuerung und die Bildbearbeitung benutzt werden. Gemäß der vorliegenden Erfindung können die Datensätze, die durch die oben aufgeführte Ausführungsform erzeugt werden, zur Visualisierung, insbesondere für diagnostische Zwecke, einem Arzt vorgelegt werden, vorzugsweise mittels einer Anzeige (9) oder eines Ausdrucks.

In einer bevorzugten Ausführungsform wird die Kieferbogenform (11a) mittels eines oder mehreren neuronaler Netze ermittelt. Die neuronalen Netze werden durch Datenpaare trainiert, welche z.B. 3D Volumen und die darin markierte Kieferbogenform (11a) umfassen, oder z.B. optischen 3D-Scan und darin markierte Kieferbogenform (11a) umfassen. Die Kieferbogenform (11b) kann manuell oder durch Bildverarbeitung unter Berücksichtigung anatomischer Merkmale automatisch markiert worden sein. Die neuronalen Netze können mit dem extraoralen Röntgen-System (1) integriert bereitgestellt werden. Alternativ können die neuronalen Netze separat bereitgestellt werden. Das extraorale Röntgensystem (1) kann mit den neuronalen Netzen lokal oder über ein Netzwerk verbunden werden. Die neuronalen Netze können durch Hardware oder Software implementiert werden.

## Patentansprüche

1. Verfahren zur Herstellung einer Panoramaaufnahme, wobei es die folgenden Schritte umfasst:
(S1) Basierend auf einer oder mehrerer vorangegangener Panoramaaufnahmen oder 3D-Aufnahmen oder eines oder mehrerer optischer 3D-Scans eines Patienten, wird die Kieferbogenform (11a) als modellbasiertes Vorwissen über die Anatomie des Patienten (10) ermittelt;
(S2) Verwendung des modellbasierten Vorwissens um eine patienten-spezifische Röntgengerätebahnkurve (12) der herzustellenden Panoramaaufnahme des Patienten (10) zu ermitteln sodass die Kieferbogenform (11a) bzw. die Stellung der Zähne (11b) optimal aufgenommen werden kann;
(S3) Durchführen der Aufnahme auf Basis der ermittelten patienten-spezifischen Röntgengerätebahnkurve (12);
(S4) Anpassung von Rekonstruktionsparametern entsprechend der ermittelten patienten-spezifischen Röntgengerätebahnkurve (12);
(S5) Verwendung des modellbasierten Vorwissens, um die patienten-spezifische Fokalkurve (14) der herzustellenden Panoramaaufnahme des Patienten (10) zu ermitteln sodass die Kieferbogenform (11a) bzw. die Stellung der Zähne (11b) optimal rekonstruiert werden kann;
(S6) Rekonstruktion der Panoramaaufnahme unter Verwendung der Aufnahmedaten, der angepassten Rekonstruktionsparameter einschließlich der ermittelten patienten-spezifischen Fokalkurve (14);
(S7) Darstellung der rekonstruierten Panoramaaufnahme,
wobei die Kieferbogenform (11a) mittels neuronaler Netze ermittelt wird, wobei die neuronalen Netze durch Datenpaare trainiert wurden, welche 3D Volumen und die darin markierte Kieferbogenform (11a) umfassen, oder optischen 3D-Scan und darin markierte Kieferbogenform (11a) umfassen.

2. Verfahren nach Anspruch 1, **dadurch gekennzeichnet, dass** die Kieferbogenform (11b) manuell oder durch Bildverarbeitung unter Berücksichtigung anatomischer Merkmale automatisch markiert worden sind.

3. Verfahren nach Anspruch 1, **dadurch gekennzeichnet, dass** bei der Rekonstruktion im Schritt (S6), der Überlapp der Kieferbogenform (11a) und einer scharfen Schicht maximal ist, wobei die patienten-spezifische Fokalkurve (14) innerhalb der scharfen Schicht liegt.

4. Computerprogramm umfassend computerlesbarer Code, das wenn es von einem computergestützten extraoralen Röntgensystem (1) ausgeführt wird dieses veranlasst, die Verfahrensschritte einer der vorhergehenden Verfahrensansprüche auszuführen.

5. Computergestütztes extraorales Röntgensystem umfassend ein Röntgengerät (2) und eine Recheneinheit (8) die zur Ausführung des Computerprogramms nach Anspruch 4 konfiguriert ist.

## Claims

1. Method for producing a panorama image, comprising the following steps:
(S1) Based on one or more previous panorama images or 3D images or one or more optical 3D scans of a patient, the shape of the jaw arch (11a) is determined as a model-based pre-knowledge of the anatomy of the patient (10);
(S2) Using the model-based pre-knowledge to determine a patient-specific X-ray trajectory (12) of the panorama image of the patient (10) to be produced so that the shape of the jaw arch (11a) or the position of the teeth (11b) can be optimally recorded;
(S3) Producing the image on the basis of the determined patient-specific X-ray trajectory (12);
(S4) Adjusting reconstruction parameters according to the determined patient-specific X-ray trajectory (12);
(S5) Using the model-based pre-knowledge to determine the patient-specific focal curve (14) of the panorama image of the patient (10) to be produced so that the shape of the jaw arch (11a) or the position of the teeth (11b) can be optimally reconstructed;
(S6) Reconstructing the panorama image using the image data, the adjusted reconstruction parameters including the determined patient-specific focal curve (14);
(S7) Representation of the reconstructed panorama image,
wherein the shape of the jaw arch (11a) is determined by means of neural networks, wherein the neural networks have been trained by data pairs comprising 3D volumes and the shape of the jaw arch (11a) marked therein, or comprising the optical 3D scan and shape of the jaw arch (11a) marked therein.

2. Method according to claim 1, **characterised in that** the shape of the jaw arch (11b) has been marked manually or automatically by means of image processing taking into account anatomical features.

3. Method according to claim 1, **characterised in that** in the reconstruction in step (S6), the overlap of the shape of the jaw arch (11a) and a sharp layer is maximal, wherein the patient-specific focal curve (14) is within the sharp layer.

4. Computer program comprising computer-readable code which, when it is executed by a computerised extraoral X-ray system (1), prompts said system to execute the method steps of one of the preceding method claims.

5. Computerised extraoral X-ray system comprising an X-ray device (2) and a computing unit (8) which is configured to execute the computer program according to claim 4.

## Revendications

1. Procédé de réalisation d'un enregistrement panoramique, comprenant les étapes suivantes :
(S1) sur la base d'un ou de plusieurs enregistrements panoramiques ou enregistrements 3D antérieurs ou d'un ou de plusieurs scans 3D optiques d'un patient, la forme de l'arc mandibulaire (11a) est déterminée en tant que connaissances antérieures basées sur le modèle de l'anatomie du patient (10) ;
(S2) utilisation des connaissances antérieures basées sur le modèle pour déterminer une courbe de trajectoire spécifique au patient de l'appareil de radiographie (12) pour l'enregistrement panoramique du patient (10) à réaliser de manière à ce que la forme de l'arc mandibulaire (11a) ou
la position des dents (11b) puisse être enregistrée de manière optimale ;
(S3) réalisation de l'enregistrement sur la base de la courbe de trajectoire spécifique au patient de l'appareil de radiographie (12) déterminée ;
(S4) adaptation des paramètres de reconstruction en fonction de la courbe de trajectoire spécifique au patient de l'appareil de radiographie (12) déterminée ;
(S5) utilisation des connaissances antérieures basées sur le modèle pour déterminer la courbe focale spécifique au patient (14) pour l'enregistrement panoramique du patient (10) à réaliser de manière à ce que la forme de l'arc mandibulaire (11a) ou la position des dents (11b) puisse être reconstruite de manière optimale ;
(S6) reconstruction de l'enregistrement panoramique en utilisant les données d'enregistrement, les paramètres de reconstruction adaptés, y compris la courbe focale spécifique au patient (14) déterminée ;
(S7) représentation de l'enregistrement panoramique reconstruit,
dans laquelle la forme de l'arc mandibulaire (11a) est déterminée au moyen de réseaux neuronaux, lesdits réseaux neuronaux ayant été entraînés au moyen de paires de données comprenant le volume 3D et la forme de l'arc mandibulaire (11a) qui y est marquée, ou comprenant un scan 3D optique et la forme de l'arc mandibulaire (11a) qui y est marquée.

2. Procédé selon la revendication 1, **caractérisé en ce que** la forme de l'arc mandibulaire (11b) a été marquée manuellement ou automatiquement par traitement d'image en tenant compte des caractéristiques anatomiques.

3. Procédé selon la revendication 1, **caractérisé en ce que**, lors de la reconstruction à l'étape (S6), le recouvrement entre la forme de l'arc mandibulaire (11a) et une couche nette est maximal, dans lequel la courbe focale spécifique au patient (14) se trouve à l'intérieur de la couche nette.

4. Programme informatique comprenant un code lisible par ordinateur qui, lorsqu'il est exécuté par un système de radiographie extra-buccale assisté par ordinateur (1), amène ce dernier à exécuter les étapes de procédé de l'une des revendications de procédé précédentes.

5. Système de radiographie extra-buccale assisté par ordinateur comprenant un appareil de radiographie (2) et une unité de calcul (8) qui est configurée pour exécuter le programme informatique selon la revendication 4.
